# EUROPÄISCHE PATENTANMELDUNG

(11) **EP 0 623 333 A2**
(43) Veröffentlichungstag der Anmeldung: **09.11.1994**
(21) Anmeldenummer: 94104047.9
(22) Anmeldetag: 16.03.1994
(51) Int. Cl.: A61F 13/20

(54) **Verfahren und Vorrichtung zur Herstellung von Tampons**

(30) Priorität: 10.04.1993 DE 4311882
(71) Anmelder: KARL RUGGLI AG, CH-8435 Fisibach (CH)
(72) Erfinder: Brinker, Alfred, Dr., D-58285 Gevelsberg (DE)
(74) Vertreter: Stenger, Watzke & Ring Patentanwälte

(57) **Zusammenfassung**

Die Erfindung betrifft ein Verfahren und eine Vorrichtung zur Herstellung von Tampons, bei denen ein im wesentlichen zylindrischer Rohling beliebiger Querschnittsform aus saugfähigen Fasern durch einen Preßvorgang mit in radialer Richtung wirkenden Kraftkomponenten zu einem Vorformling (V) mit einem Kern höherer Verdichtung und einer Randzone aus axial verlaufenden Rippen und zwischen den Rippen verlaufenden Längsnuten geformt wird, der anschließend durch eine zweiten Preßvorgang auf einen kleineren Durchmesser komprimiert wird. Um eine Leistungssteigerung zu erzielen, wird der Vorformling (V) nach der ersten Pressung ohne zusätzliche Verdichtung in eine Zwischenposition überführt, aus der er dem zweiten Preßvorgang zugeführt wird. Diese Zwischenposition wird durch eine Buchse (3) gebildet, die zwischen einer hinter dem Preßwerkzeug (1) liegenden Aufnahmestellung und einer vor dem versetzt zum Preßwerkzeug (1) angeordneten Formwerkzeug (4) liegenden Übergabestellung beweglich ist. In der Übergabestellung wirkt mit der Buchse (3) ein Preßstößel (6) zusammen, dessen Durchmesser (S2) etwa dem Austrittsdurchmesser (4b) des Formwerkzeugs (4) entspricht.

## Beschreibung

Die Erfindung betrifft ein Verfahren und eine Vorrichtung zur Herstellung von Tampons, bei denen ein im wesentlichen zylindrischer Rohling beliebiger Querschnittsform aus saugfähigen Fasern durch einen Preßvorgang mit in radialer Richtung wirkenden Kraftkomponenten zu einem Vorformling mit einem Kern höherer Verdichtung und einer Randzone aus axial zur Tamponachse verlaufenden Rippen und zwischen den Rippen verlaufenden Längsnuten geformt wird, der anschließend durch einen zweiten Preßvorgang auf einen kleineren Durchmesser komprimiert wird.

Ein derartiges Verfahren zur Herstellung von Tampons ist aus der DE 39 34 153 C2 bekannt. Bei diesem bekannten Verfahren wird der Rohling durch zwei Gruppen von zentripetal zur Rohlingslängsachse in zwei Stufen zustellbaren Preßbacken mit die Längsnuten und damit auch die Rippen erzeugenden Preßschneiden zum Vorformling geformt. Der Vorformling wird anschließend mittels eines Stößels in axialer Richtung aus den in geschlossener Stellung verharrenden oder geringfügig geöffneten Preßbacken durch ein unmittelbar benachbartes Formwerkzeug gepreßt. Dieses Formwerkzeug ist konisch mit einem dem Außendurchmesser des Vorformlings entsprechenden Eintrittsdurchmesser und einem dem endgültigen Außendurchmesser des Tampons entsprechenden Austrittsdurchmesser ausgeführt.

Da der Durchmesser des Stößels kleiner sein muß als die in den Vorformling hineinragenden Preßschneiden der Preßbacken, erfaßt die Stirnfläche des Stößels nur den Kern des Vorformlings. Beim Ausschieben dieses Vorformlings aus den geschlossenen bzw. geringfügig geöffneten Preßbacken sind jedoch nicht nur die zwischen der zerklüfteten Mantelfläche des Vorformlings und den mit Preßschneiden versehenen Preßbacken entstehenden Losbrechkräfte und bei der sich anschließenden Bewegung Reibungskräfte gegenüber der geschlossenen Form zu überwinden, sondern bereits nach einem geringen Hub des Stößels noch zusätzliche Verformungskräfte durch die konische Form des unmittelbar nachgeschalteten Formwerkzeugs. Es entsteht somit eine sehr hohe, ausschließlich auf den Kern des Vorformlings ausgeübte Kraft, die zu einer hohen Flächenpressung führt und zu einer Begrenzung der Auspreßgeschwindigkeit zwingt, wenn unzulässige Verformungen des Vorformlings bzw. des entstehenden Tampons vermieden werden sollen. Bei einer zu hohen Auspreßgeschwindigkeit würde der harte Kern des Tampons in axialer Richtung gegenüber dem durch Rippen und Längsnuten gebildeten Randbereich verschoben werden. Die Leistung des bekannten Verfahrens ist deshalb auf eine mittlere Ausschiebegeschwindigkeit von ca. 0,6 m/sek. begrenzt.

Der Erfindung liegt die **Aufgabe** zugrunde, die Leistung des bekannten, eingangs beschriebenen Verfahrens zur Herstellung von Tampons zu steigern, ohne an der Grundkonzeption der Preßvorgänge Änderungen vorzunehmen.

Die **Lösung** dieser Aufgabenstellung durch die Erfindung ist dadurch gekennzeichnet, daß der Vorformling nach der ersten Pressung ohne zusätzliche Verdichtung in eine Zwischenposition überführt wird, aus der er dem zweiten Preßvorgang zugeführt wird.

Durch die erfindungsgemäße Weiterbildung des bekannten Verfahrens ergibt sich der Vorteil, daß beim Ausschieben des Vorformlings aus der geschlossenen oder teilgeöffneten Form lediglich die Losbrech- und Reibungskräfte zu überwinden sind, wozu die geringe Stirnfläche des Stößels ausreicht. Aus der Zwischenposition wird der Vorformling anschließend in einem zweiten Arbeitsgang dem konischen Formwerkzeug zugeführt, wozu ein zweiter Stößel mit einem größeren Durchmesser verwendet werden kann, so daß die für den zweiten Preßvorgang aufzuwendenden Verformungskräfte innerhalb des konischen Formwerkzeuges mittels einer größeren Stirnfläche auf den Vorformling übertragen werden können, wobei die Stirnfläche dieses zweiten Stößels nicht nur am Kern, sondern auch an einem Teil des Randbereiches des Vorformlings angreift, weil der Außendurchmesser des zweiten Stößels nahezu dem Austrittsdurchmesser des konischen Formwerkzeuges entsprechen kann.

Die erfindungsgemäße Trennung des Ausschiebens des Vorformlings aus der geschlossenen bzw. teilgeöffneten Preßform von der anschließenden Verformung im konischen Formwerkzeug ermöglicht trotz der Einschaltung einer Zwischenposition eine erhebliche Leistungssteigerung um etwa 50 %, ohne daß sich komplizierte Bewegungsabläufe ergeben.

Die erfindungsgemäße Vorrichtung zur Herstellung von Tampons aus einem im wesentlichen zylindrischen Rohling beliebiger Querschnittsform aus saugfähigen Fasern umfaßt in bekannter Weise ein erstes, aus mehreren, mindestens teilweise mit Preßschneiden versehenen Preßbacken bestehendes Preßwerkzeug, aus dem der Vorformling mittels eines Stößels in axialer Richtung ausgeschoben wird, und ein zweites Formwerkzeug, dessen Eintrittsdurchmesser dem Außendurchmesser des Vorformlings und dessen Austrittsdurchmesser dem Durchmesser des fertigen Tampons entspricht.

Die erfindungsgemäße Weiterbildung ist gekennzeichnet durch eine mit einem dem Außendurchmesser des Vorformlings entsprechenden Innendurchmesser ausgeführte Buchse, die zwischen einer hinter dem Preßwerkzeug liegenden Aufnahmestellung und einer vor dem versetzt zum Preßwerkzeug angeordneten Formwerkzeug liegenden Übergabestellung beweglich ist, und durch einen zweiten, mit der in der Übergabestellung befindlichen Buchse zusammenwirkenden Preßstößel, dessen Durchmesser etwa dem Austrittsdurchmesser des zweiten Formwerkzeugs entsprechend ausgebildet ist.

Bei dem innerhalb der erfindungsgemäßen Vorrichtung verwendeten Preßwerkzeug können Preßbacken verwendet werden, die entsprechend der aus der DE 39 34 153 C2 bekannten Vorrichtung ausschließlich in radialer Richtung bewegt werden. Alternativ können auch Preßbacken Verwendung finden, die gemäß der aus der DE 26 23 368 C3 bekannten Vorrichtung auf einer bogenförmigen Bahn tangential zu einem gedachten Kreis mit vorgegebenem Radius bewegt werden. In beiden Fällen werden Preßkräfte mit radialer Kraftkomponente auf den Rohling ausgeübt, die den Rohling zu einem Vorformling mit axial zur Tamponachse verlaufenden Rippen und zwischen den Rippen verlaufenden Längsnuten verformen.

Auf der Zeichnung ist ein Ausführungsbeispiel der erfindungsgemäßen Vorrichtung schematisch dargestellt, anhand dessen auch das erfindungsgemäße Verfahren erläutert werden wird. Es zeigen:
- Fig. 1: eine schematische Darstellung der Hauptteile der Vorrichtung im Längsschnitt,
- Fig. 2: einen Querschnitt durch das erste Preßwerkzeug gemäß der Schnittlinie II - II in Fig. 1,
- Fig. 3: einen Querschnitt durch die Buchse gemäß der Schnittlinie III - III in Fig. 1 und
- Fig. 4: einen Querschnitt durch den fertigen, in einer Aufnahmebuchse liegenden Tampon gemäß der Schnittlinie IV - IV in Fig. 1.

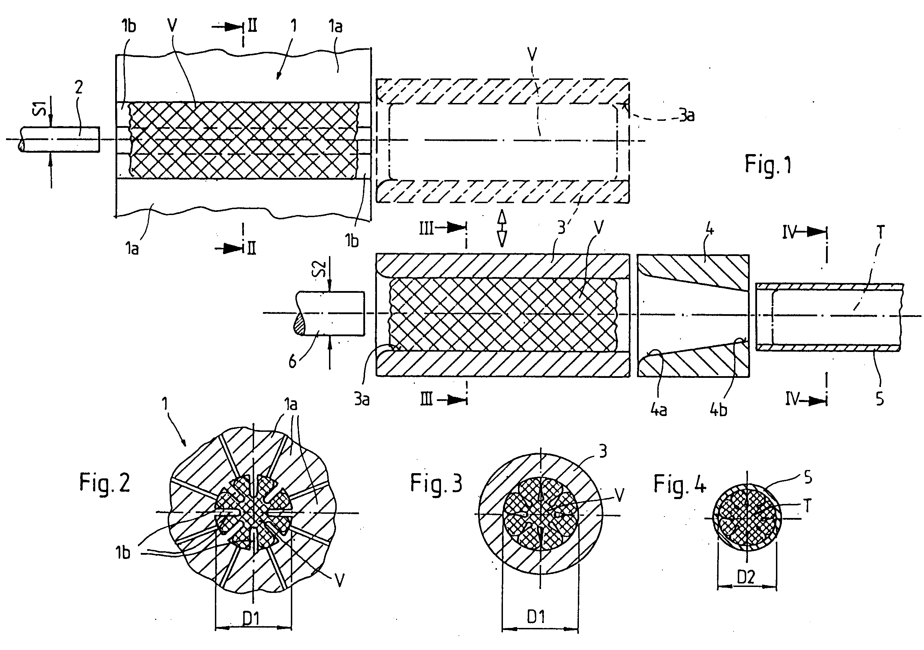

Die auf der Zeichnung nur schematisch dargestellte Vorrichtung umfaßt ein Preßwerkzeug 1, das beim Ausführungsbeispiel aus acht in radialer Richtung beweglichen Preßbacken 1a besteht, die jeweils mit einer in axialer Richtung verlaufenden Preßschneide 1b versehen sind. Wenn die Preßbacken 1a dieses Preßwerkzeuges 1 nach dem Einführen eines aus saugfähigen Fasern bestehenden Rohlings beliebiger Querschnittsform in die in Fig. 2 erkennbare Endstellung geschlossen worden sind, ergibt sich ein Vorformling V mit einem Kern höherer Verdichtung und einer Randzone aus axial verlaufenden Rippen und zwischen den Rippen verlaufenden Längsnuten, wie deutlich aus Fig. 2 hervorgeht.

Zum Ausschieben dieses Vorfromlings V aus dem Preßwerkzeug 1 ist ein Ausschiebestößel 2 vorgesehen, dessen Durchmesser S1 geringfügig kleiner ist als der Abstand zwischen einander gegenüberliegenden Preßschneiden 1b bei in der Endstellung befindlichen Preßbacken 1a.

In Ausschieberichtung hinter dem Preßwerkzeug 1 ist eine Buchse 3 angeordnet, deren Innendurchmesser 3a geringfügig größer als der Außendurchmesser des Vorformlings V ist. Diese Buchse 3 ist zwischen ihrer hinter dem Preßwerkzeug 1 liegenden Aufnahmestellung, die in Fig. 1 gestrichelt dargestellt ist, und einer Übergabestellung beweglich, die in Fig. 1 mit ausgezogenen Linien dargestellt ist und in der die Buchse 3 vor einem Formwerkzeug 4 mit konischer Innenkontur liegt. Der Eintrittsdurchmesser 4a des Formwerkzeuges 4 ist geringfügig größer als der Außendurchmesser des Vorformlings V. Der Austrittsdurchmesser 4b bestimmt den Außendurchmesser des fertigen Tampons T, der strichpunktiert in einer Aufnahmebuchse 5 liegend in Fig. 1 eingezeichnet ist.

Um diesen Tampon T aus dem Vorformling V herzustellen, wird der in der Buchse 3 liegende Vorformling V gemäß Fig. 1 mit Hilfe eines Preßstößels 6 aus der vor dem Formwerkzeug 4 liegenden Buchse 3 heraus und durch das Formwerkzeug 4 hindurchgeschoben. Hierbei verringert sich der Außendurchmesser des Vorformlings V auf den Austrittsdurchmesser 4b des Formwerkzeugs 4. Um diesen Außendurchmesser für den fertigen Tampon T beizubehalten, ist es erforderlich, den Tampon T für die Weiterverarbeitung der Aufnahmebuchse 5 zuzuführen.

Während der Außendurchmesser S1 des Stößels 2 durch die Lage der Preßschneiden 1b der in geschlossener oder geringfügig geöffneten Stellung befindlichen Preßbacken 1a bestimmt wird, kann der Durchmesser S2 des Preßstößels 6 erheblich größer ausgeführt werden, da er etwa dem Austrittsdurchmesser 4b des Formwerkzeuges 4 entsprechend ausgeführt werden kann. Der die Verformungskräfte innerhalb des Formwerkzeuges 4 auf den Vorformling V aufbringende Preßstößel 6 kann somit mit erheblich größerer Stirnfläche ausgeführt werden als der Ausschiebestößel 2, der die Losbrech- und Reibungskräfte aufbringen muß, die erforderlich sind, um den im Preßwerkzeug 1 befindlichen Vorformling V aus den geschlossenen Preßbacken 1a herauszuschieben, wobei insbesondere die in die Außenkontur des Vorformlings V eingreifenden Preßschneiden 1b hohe Losbrech- und Reibungskräfte hervorrufen.

Ein Vergleich der Fig. 2,3 und 4 zeigt, daß der Außendurchmesser D1 des Vorformlings V bei der Überführung aus dem Preßwerkzeug 1 in die Buchse 3 unverändert bleibt und daß eine Reduzierung auf den Außendurchmesser D2 des Tampons T erst nach dem Ausschieben des Vorformlings V aus der Buchse 3 durch das Formwerkzeug 4 erfolgt.

### Bezugszeichenliste:

- V: Vorformling
- T: Tampon
- D1: Außendurchmesser Vorformling
- D2: Außendurchmesser Tampon
- S1: Durchmesser Ausschiebestößel
- S2: Durchmesser Preßstößel
- 1: Preßwerkzeug
- 1a: Preßbacke
- 1b: Preßschneide
- 2: Ausschiebestößel
- 3: Buchse
- 3a: Innendurchmesser
- 4: Formwerkzeug
- 4a: Eintrittsdurchmesser
- 4b: Austrittsdurchmesser
- 5: Aufnahmebuchse
- 6: Preßstößel

## Patentansprüche

1. Verfahren zur Herstellung von Tampons, bei dem ein im wesentlichen zylindrischer Rohling beliebiger Querschnittsform aus saugfähigen Fasern durch einen Preßvorgang mit in radialer Richtung wirkenden Kraftkomponenten zu einem Vorformling mit einem Kern höherer Verdichtung und einer Randzone aus axial zur Tamponachse verlaufenden Rippen und zwischen den Rippen verlaufenden Längsnuten geformt wird, der anschließend durch einen zweiten Preßvorgang auf einen kleineren Durchmesser komprimiert wird,
**dadurch gekennzeichnet,**
daß der Vorformling (V) nach der ersten Pressung ohne zusätzliche Verdichtung in eine Zwischenposition überführt wird, aus der er dem zweiten Preßvorgang zugeführt wird.

2. Vorrichtung zur Herstellung von Tampons aus einem im wesentlichen zylindrischen Rohling beliebiger Querschnittsform aus saugfähigen Fasern mit einem ersten, aus mehreren, mindestens teilweise mit Preßschneiden versehenen Preßbacken bestehenden Preßwerkzeug, aus dem der Vorformling mittels eines Stößels in axialer Richtung ausgeschoben wird, und mit einem zweiten Formwerkzeug, dessen Eintrittsdurchmesser dem Außendurchmesser des Vorformlings und dessen Austrittsdurchmesser dem Durchmesser des fertigen Tampons entspricht,
**gekennzeichnet durch**
eine mit einem dem Außendurchmesser (D1) des Vorformlings (V) entsprechenden Innendurchmesser (3a) ausgeführte Buchse (3), die zwischen einer hinter dem Preßwerkzeug (1) liegenden Aufnahmestellung und einer vor dem versetzt zum Preßwerkzeug (1) angeordneten Formwerkzeug (4) liegenden Übergabestellung beweglich ist, und durch einen zweiten, mit der in der Übergabestellung befindlichen Buchse (3) zusammenwirkenden Preßstößel (6), dessen Durchmesser (S2) etwa dem Austrittsdurchmesser (4b) des Formwerkzeugs (4) entsprechend ausgebildet ist.
